(19) Europäisches Patentamt
European Patent Office
Office européen des brevets

(11) Veröffentlichungsnummer: **0 038 480**
**B1**

# (12) EUROPÄISCHE PATENTSCHRIFT

(45) Veröffentlichungstag der Patentschrift: **21.09.83**

(51) Int. Cl.³: **C 07 C 49/245,** C 07 C 49/255, C 07 C 45/00

(21) Anmeldenummer: **81102697.0**

(22) Anmeldetag: **09.04.81**

(54) **Verfahren zur Herstellung von 1-(4-Hydroxy-phenyl)-butan-3-on sowie neue Zwischenprodukte dieses Verfahrens.**

(30) Priorität: **22.04.80 DE 3015359**

(43) Veröffentlichungstag der Anmeldung: **28.10.81 Patentblatt 81/43**

(45) Bekanntmachung des Hinweises auf die Patenterteilung: **21.09.83 Patentblatt 83/38**

(84) Benannte Vertragsstaaten: **CH DE FR GB LI NL**

(56) Entgegenhaltungen:
**DE-B-1 098 951**
**DE-C-702 894**
**Houben-Weyl, «Methoden der organischen Chemie», 4. Auflage (1976) Band VI/1c, Seite 316, Absatz 2**

(73) Patentinhaber: **BASF Aktiengesellschaft, Carl-Bosch-Strasse 38, D-6700 Ludwigshafen (DE)**

(72) Erfinder: **Hoffmann, Werner, Dr., Ringstrasse 11 C, D-6701 Neuhofen (DE)**
Erfinder: **Degner, Dieter, Dr., Kurpfalzstrasse 8, D-6701 Dannstadt-Schauernheim (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 1-(4-Hydroxy-phenyl)-butan-3-on sowie neue Zwischenprodukte dieses Verfahrens

Die Erfindung betrifft ein Verfahren zur Herstellung von 1-(4-Hydroxy-phenyl)-butan-3-on sowie als neue Zwischenprodukte für dieses Verfahren 1-(4-tert.-Alkoxy-phenyl)-butan-3-one bzw. -but-1-en-3-one der allgemeinen Formel III

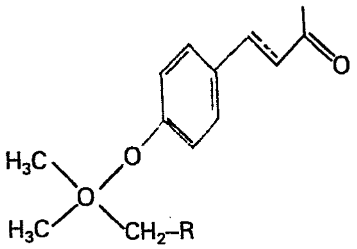

(III),

in der R für H oder $CH_3$-, vorzugsweise für H, steht und die gestrichelte Linie eine weitere C-C-Bindung bedeuten kann.

1-(4-Hydroxy-phenyl)-butan-3-on ist ein begehrter natürlicher Aromastoff, Himbeerketon oder Frambion genannt. Es hat daher nicht an Versuchen gefehlt, diese Verbindung vorteilhaft herzustellen.

Die bisher wichtigsten Herstellungsverfahren beinhalten eine Alkylierung von Phenol mit Methylvinylketon bzw. dessen Derivaten in Gegenwart von sauren Katalysatoren (vgl. DE-PS 2 145 308). Nachteilig an diesen Verfahren sind einerseits unbefriedigende Ausbeuten sowie die Notwendigkeit zu aufwendigen Reinigungsoperationen zur Erzielung der erforderlichen Aromenqualität, da es durch die hohe Reaktivität der Ausgangsmaterialien zur Bildung grösserer Mengen an Nebenprodukten kommt.

Weiterhin ist es bekannt 4-Hydroxy-benzaldehyd mit Aceton zu 4-Hydroxy-benzalaceton zu kondensieren, welches anschliessend zu 4-(-Hydroxy-phenyl)-butan-2-on hydriert werden kann (vgl. J. Am. Chem. Soc. 70 (1948) S. 3360). Der als Ausgangsverbindung für diesen zweistufigen Prozess notwendige 4-Hydroxy-benzaldehyd ist jedoch eine nur schwer zugängliche und daher eine sehr teure Substanz. Ferner werden bei diesem Verfahren insgesamt nur unbefriedigende Ausbeuten an 4-(4-Hydroxy-phenyl)-butan-2-on erzielt.

Gemäss der britischen Patentschrift 1 094 417 wird das Himbeerketon dadurch erhalten, dass man p-Methoxy-benzylchlorid in Gegenwart von Kaliumcarbonat mit Acetessigsäureethylester umsetzt und das so erhaltene 1-(p-Methoxy-phenyl)-butan-3-on mit konzentrierter Bromwasserstofflösung spaltet. Wie Beispiel 2 dieses Patentes zeigt, ist die Spaltung des Methylethers mit HBr unvollständig und dadurch die Isolierung des gewünschten Himbeerketons erheblich erschwert. Darüber hinaus erfordern die Handhabung des Bromwasserstoffs und des frei werdenden Methylbromids einen hohen apparativen Aufwand.

Es war daher die Aufgabe der Erfindung, ein Verfahren zu entwickeln, gemäss dem das Himbeerketon auf einfache Weise, aus gut zugänglichen Ausgangsverbindungen in möglichst reiner Form und mit guten Ausbeuten erhalten wird.

Gegenstand der Erfindung ist ein Verfahren zur Herstellung von 1-(4-Hydroxy-phenyl)-butan-3-on (I), das dadurch gekennzeichnet ist, dass man

A. den entsprechenden 4-tert.-Alkoxy-benzaldehyd der allgemeinen Formel II

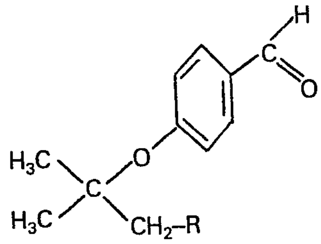

(II),

in der R für H oder $CH_3$ steht, unter alkalischen Bedingungen mit Aceton zu dem neuen 1-(4-tert.-Alkoxy-phenyl)-but-1-en-3-on der allgemeinen Formel IIIa

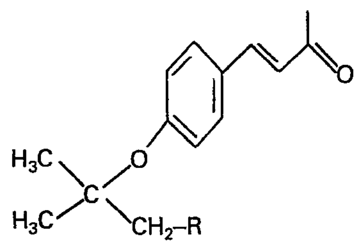

(IIIa)

kondensiert,

B. dieses während oder nach dieser Umsetzung zu dem neuen 1-(4-tert.-Alkoxy-phenyl)-butan-3-on der Formel IIIb

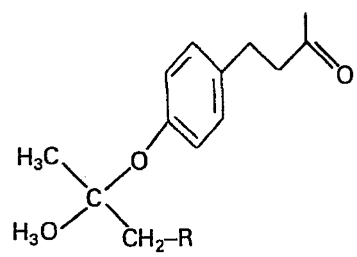

(IIIb)

hydriert und

C. hieraus bei 40 bis 150 °C in Gegenwart katalytischer Mengen einer Säure Isobuten bzw. 2-Methyl-1 (2)-buten abspaltet sowie die neuen Zwischenprodukte für dieses Verfahren mit der allgemeinen Formel III.

Überraschenderweise werden bei dem erfindungsgemässen Herstellungsverfahren über die neuen Zwischenprodukte alle Nachteile der bekannten Verfahren weitgehend überwunden. So sind z.B. die als Ausgangsmaterial benötigten 4-tert.-Alkoxy-benzaldehyde der Formel II neuerdings leicht und in guten Ausbeuten erhältlich. Ihre Herstellung kann gemäss der nicht vorveröffentlichten DE-OS 29 35 398 durch elektrochemische Oxydation von p-tert.-Butoxy-toluol in Gegenwart eines Alkohols und anschliessende Acetalspaltung erfolgen.

Im Gegensatz zu den Ausgangsstoffen und Zwischenprodukten bei der Kondensation von p-Hydroxy-benzaldehyd (bei der zudem noch mindestens molare Mengen an Alkali durch Phenolatbildung verloren gehen) sind die neuen Ausgangsmaterialien und Zwischenprodukte keine Feststoffe, sondern Flüssigkeiten, wodurch der Arbeitsablauf insbesondere in technischem Massstab wesentlich vereinfacht wird. Die neuen Zwischenprodukte IIIa und IIIb können durch Destillation auf einfache Weise in 99%iger Reinheit erhalten werden. Die tert. Alkylgruppen spalten unter den Destillationsbedingungen nicht ab.

Sowohl die Kondensation mit Aceton als auch die Hydrierung gelingen auf einfache Weise und mit guten Ausbeuten. Besonders vorteilhaft gestaltet sich die Abspaltung der Schutzgruppe des Phenols, die mit Spuren von Säuren in wässriger Suspension vorgenommen werden kann und zu einem kristallinen Produkt führt, welches durch einmaliges Umkristallisieren eine Reinheit von 99% aufweist.

Die Umsetzung der 4-tert.-Alkoxy-benzaldehyde der Formel II mit Aceton erfolgt in an sich bekannter Weise unter basischen Reaktionsbedingungen. Pro Mol Aldehyd kommen im allgemeinen 1 bis 10, insbesondere 3 bis 6 Mol Aceton zum Einsatz. Als Katalysatoren eignen sich Alkali- und Erdalkalihydroxide sowie Alkali- und Erdalkalialkoholate. Man setzt pro Mol Aldehyd 0,0001 bis 0,1, insbesondere 0,001 bis 0,01 Mol des Katalysators ein. Die Umsetzung kann mit oder ohne inerte Lösungs- bzw. Verdünnungsmittel, diskontinuierlich oder kontinuierlich durchgeführt werden. Als günstig haben sich Reaktionstemperaturen zwischen 0 und 50 °C, insbesondere zwischen 15 und 35 °C erwiesen. Der Reaktionsverlauf kann dünnschicht- und gaschromatographisch verfolgt werden. Nach Beendigung der Umsetzung wird der Katalysator mit einer anorganischen oder organischen Säure neutralisiert und das erhaltene 1-(4-tert.-Alkoxy-phenyl)-but-1-en-3-on der Formel IIIa nach üblicher Aufarbeitung durch Destillation isoliert.

Die Hydrierung der 1-Buten-3-one der Formel IIIa kann in üblicher Weise ohne oder in einem inerten Lösungsmittel wie Methanol, Essigester oder Tetrahydrofuran erfolgen. Als Katalysatoren sind übliche Hydrierkatalysatoren geeignet. Vorzugsweise werden Palladium- oder Platinkatalysatoren auf Trägern wie Aktivkohle, Kieselgel oder Aluminiumoxid verwendet.

Die Reaktionstemperatur kann zwischen Raumtemperatur und 150 °C, der Wasserstoffdruck zwischen Normaldruck und 50 bar liegen. Die Isolierung der 1-(4-tert.-Alkoxy-phenyl)-butan-3-one der Formel IIIb erfolgt im allgemeinen durch Filtration und anschliessende Destillation.

Die direkte Umsetzung der Benzaldehyde der Formel II mit Aceton und Wasserstoff zu den Butanonen IIIb wird mit Hilfe von Mischkatalysatoren vorgenommen. Geeignete Katalysatoren sind hierbei Zinkoxid, Aluminiumoxid und Aktivkohle, die mit Kupfer, Nickel, Kobalt, Palladium oder Platin dotiert sind. Mit Vorteil arbeitet man auch mit einem Katalysatorsystem, das einerseits ein Oxid oder Salz eines seltenen Erdmetalls und andererseits ein Metall der Gruppe VIII des Periodensystems der Elemente enthält, wie es in der DE-PS 26 15 308 beschrieben ist. Die Reaktionstemperaturen liegen zwischen 150 bis 250 °C bei Drucken von 10 bis 50 bar.

Die Abspaltung der Schutzgruppe des Phenols kann mit wässrigen Säuren wie Schwefelsäure, Phosphorsäure, Salzsäure, Ameisensäure oder Essigsäure vorgenommen werden. Es genügen hiervon katalytische Mengen, d.h. Mengen von 0,001 bis 0,5 Mol pro Mol IIIb. Die Abspaltung wird im allgemeinen bei Temperaturen von 40 bis 150 °C, vorzugsweise 50 bis 110 °C vorgenommen. Lösungsmittel sind im allgemeinen nicht erforderlich. Mit Vorteil arbeitet man in wässriger Suspension. So kann man nach Beendigung der Etherspaltung das Verfahrensprodukt in kristalliner Form (ca. 95%ig) isolieren. Es kann dann durch einmalige Umkristallisation aus Äthanol/Wasser oder Methyl-tert.-Butylether auf ca. 99% gereinigt werden.

Das bei der Spaltung freiwerdende Isobutylen bzw. 2-Methyl-1(2)-buten kann wieder zur Herstellung von p-tert.-Alkoxy-toluol verwendet werden.

Mit Hilfe des erfindungsgemässen Verfahrens kann das als Aromastoff begehrte 1-(4-Hydroxyphenyl)-butan-3-on auf einfache Weise, in reiner Form und guten Ausbeuten aus gut zugänglichen Ausgangsstoffen hergestellt werden. Die neuen Verbindungen IIIa und IIIb ermöglichen erst das vorteilhafte Verfahren. Weiterhin eignen sich die Butene IIIa aufgrund ihrer UV-Absorption zwischen 300 und 310 nm auch als Lichtschutzmittel mit Filterwirkung im UV-B-Bereich (Sonnenschutzmittel).

## Beispiel 1

a) Herstellung von 1-(4-tert.-Butoxy-phenyl)-but-1-en-3-on

Zu einer Mischung aus 290 g (5 Mol) Aceton und 30 ml einer 0,5%igen wässrigen Natronlauge werden unter Rühren bei 20 bis 25 °C tropfenweise innerhalb von 15 Minuten 89 g (0,5 Mol) p-tert.-Butoxy-benzaldehyd gegeben. Nach Beendigung des Zulaufs muss der pH-Wert noch mindestens 10 sein, andernfalls muss noch Natronlauge nachgegeben werden. Die Umsetzung ist nach ca. 5 Stunden beendet, was durch Dünnschicht- oder Gaschromatographie nachgewiesen werden kann. Anschliessend versetzt man die Lösung mit Essigsäure bis zum Neutralpunkt, destilliert unter vermindertem Druck das überschüssige Aceton ab und nimmt das Reaktionsprodukt in Ether auf. Die Etherlösung wird mit Wasser salzfrei gewaschen, der Ether abdestilliert und das Reaktionsprodukt fraktioniert, wobei 88 g Hauptlauf erhalten werden. Das entspricht einer Ausbeute von 81% der Theorie.

Siedepunkt: 117 – 120 °C bei 0,01 mbar; $n_D^{25} = 1{,}5689$; UV-Spektrum: $E_{1cm}^{1\%}$ bei R max 305 nm: 896.

b) Herstellung von 1-(4-tert.-Butoxy-phenyl)-butan-3-on

109 g (0,5 Mol) eines gemäss a) hergestellten 1-(4-tert.-Butoxy-phenyl)-but-1-en-3-ons werden mit 3 g eines 0,5%igen Palladium/Aluminiumoxid-Katalysators in Pulverform versetzt und bei 100 °C und 10 bar Wasserstoffdruck bis zur Beendigung der Wasserstoffaufnahme hydriert. Danach wird vom Katalysator abfiltriert und das Reaktionsprodukt fraktioniert, wobei 101 g Hauptlauf erhalten werden (Ausbeute: 92% der Theorie).

Siedepunkt: 114–116 °C bei 0,01 mbar; $n_D^{25}$ = 1,4981.

c.) Herstellung von 1-(4-Hydroxy-phenyl)-butan-3-on

100 g (0,45 Mol) 1-(4-tert. -Butoxy-phenyl)-butan-3-on werden in 250 ml 2n Schwefelsäure suspendiert und unter kräftigem Rühren ca. 3 Stunden unter Rückfluss zum Sieden erhitzt. Der Reaktionsverlauf kann durch Messen des freiwerdenden Isobutylens verfolgt werden. Nach Beendigung der Umsetzung wird das Reaktionsgemisch auf Raumtemperatur abgekühlt und mit verdünnter Natronlauge auf pH 6 eingestellt, wonach das Verfahrensprodukt in ca. 95%iger Reinheit auskristallisiert. Es wird durch Filtrieren oder Zentrifugieren isoliert (73 g 95%ig; Ausbeute 93%) und aus Wasser/Ethanol umkristallisiert

Festpunkt: 83 – 85 °C.

Beispiel 2

Herstellung von 1-(4-tert.-Butoxy-phenyl)-butan-3-on

In einem 300 ml Rührautoklav werden 58 g (1 Mol) Aceton und 36 g (0,2 Mol) p-tert.-Butoxybenzaldehyd vorgelegt, mit 5 g Zinkoxid und 1 g Palladium auf Aktivkohle (1 %ig) versetzt und unter kräfgigem Rühren bei 20 bar Wasserstoffdruck auf 160 °C erhitzt. Nach 2 Stunden Reaktionszeit lässt man abkühlen, filtriert vom Katalysator ab und unterwirft das Reaktionsgemisch einer fraktionierten Destillation. Man erhält 19 g 1-(4-tert.-Butoxy-phenyl)-butan-3-on (Ausbeute: 43%).

Beispiel 3

a) Analog Beispiel 1a wird aus 290 g (5 Mol) Aceton, 30 ml einer 0,5%igen wässrigen Natronlauge und 96 g (0,5 Mol) p-tert.-Amyloxy-benzaldehyd das 1-(4-tert.-Amyloxy-phenyl)-but-1-en-3-on hergestellt.

Kp = 135 °C bei 0,01 mbar; $n_D^{25}$ = 1,5648;

UV-Spektrum: $E_{1\,cm}^{1\%}$ = 886 bei R max 311 nm (Isopropanol)

b) Analog Beispiel 1b wird aus 116 g (0,5 Mol) eines gemäss Beispiel 3a) hergestellten 1-(4-tert.-Amyloxy-phenyl)-but-1-en-3-ons 1-(4-tert.-Amyloxy-phenyl)-butan-3-on hergestellt.

Kp = 128 – 129 °C bei 0,01 mbar
$n_D^{25}$ = 1,4894.

**Patentansprüche**

1. Verfahren zur Herstellung von 1-(4-Hydroxyphenyl)-butan-3-on (I), dadurch gekennzeichnet, dass man

A. den entsprechenden 4-tert.-Alkoxy-benzaldehyd der allgemeinen Formel II

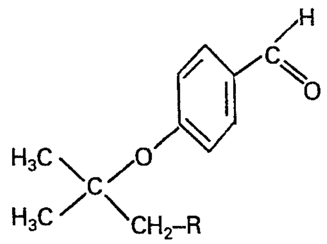

(II),

in der R für H oder $CH_3$ steht, unter alkalischen Bedingungen mit Aceton zu dem neuen 1-(4-tert.-Alkoxy-phenyl)-but-1-en-3-on der allgemeinen Formel IIIa

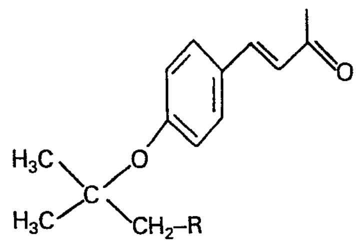

(IIIa)

kondensiert,

B. dieses während oder nach dieser Umsetzung zu dem neuen 1-(4-tert.-Alkoxy-phenyl)-butan-3-on der Formel IIIb

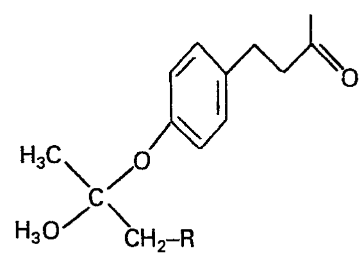

(IIIb)

hydriert und

C. hieraus bei 40 bis 150 °C in Gegenwart katalytischer Mengen einer Säure Isobuten bzw. 2-Methyl-1(2)-buten abspaltet.

2. 1-(4-tert.-Alkoxy-phenyl)-butan-3-one bzw. -but-1-en-3-one der allgemeinen Formel III

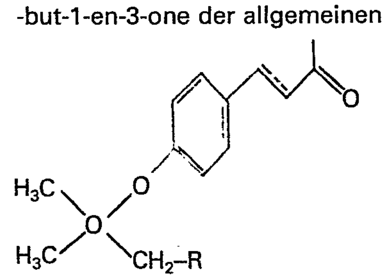

(III),

in der R für H oder $CH_3$ steht und die gestrichelte Linie eine weitere C-C-Bindung bedeuten kann.

**Revendications**

1. Procédé de préparation de la 1-(4-hydroxyphényl)-butane-3-one (I) caractérisé en ce que

A) par condensation du 4-tert-alcoxy-benzaldéhyde correspondant de formule générale II

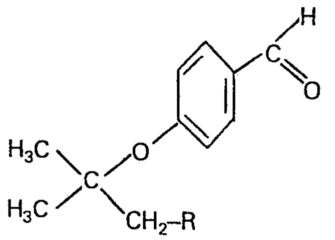

(II),

dans laquelle R représente H ou $CH_3$, avec l'acétone en milieu alcalin, on forme la nouvelle 1-(4-tert-alcoxy-phényl)-buta-1-ène-3-one de formule générale IIIa

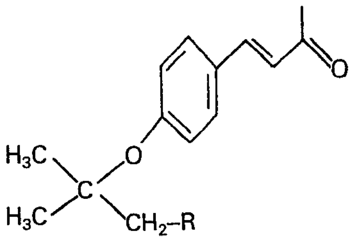

(IIIa)

B) on hydrogène cette dernière, durant ou après cette réaction, en la nouvelle 1-(4-tert-alcoxy-phényl)-butane-3-one de formule générale IIIb

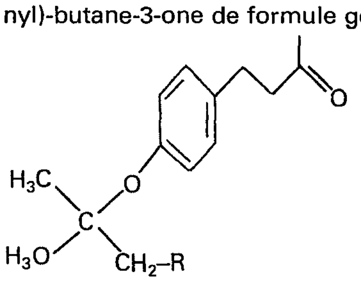

(IIIb)

et

C) on sépare de cette dernière, à une température de 40 à 150 °C, en présence de quantités catalytiques d'un acide, l'isobutène ou le 2-méthyl-1(2)-butène respectivement.

2. Les 1-(4-tert-alcoxy-phényl)-butane-3-ones et -buta-1-ène-3-ones de formule générale III

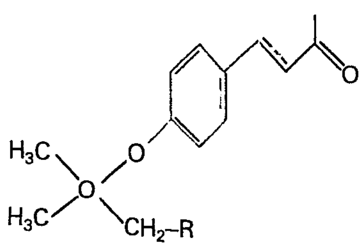

(III),

dans laquelle R représente H ou $CH_3$ et le trait interrompu peut représenter une autre liaison C-C.

**Claims**

1. A process for the preparation of 1-4-hydroxy-phenyl)-butan-3-one (I), wherein

A. a corresponding 4-tert.-alkoxy-benzaldehyde of the general formula II

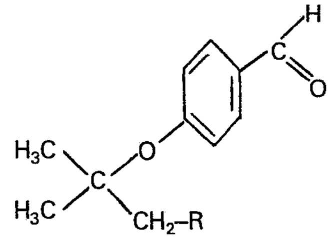

(II),

where R is H or $CH_3$, is condensed with acetone under alkaline conditions to give the novel 1-(4-tert.-alkoxy-phenyl)-but-1-en-3-one of the general formula IIIa

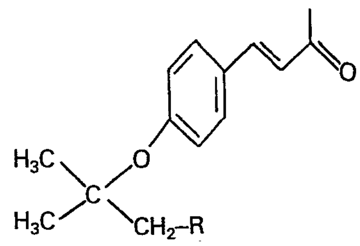

(IIIa)

B. the latter is hydrogenated, during of after this condensation, to give the novel 1-(4-tert.-alkoxy-phenyl)-butan-3-one of the formula IIIb

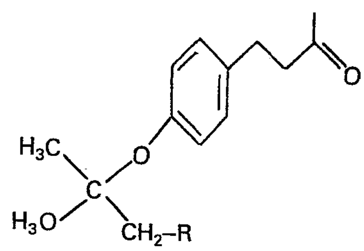

(IIIb)

and

C. isobutene or 2-methyl-but-1(2)-ene is eliminated therefrom, at from 40 to 150 °C, in the presence of a catalytic amount of an acid.

2. 1-(4-tert.-alkoxy-phenyl)-butan-3-one or -but-1-en-3-one of the general formula III

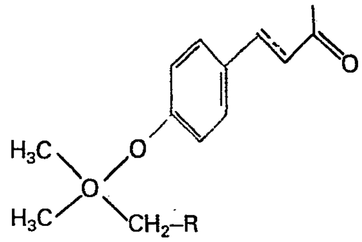

(III),

where R is H or $CH_3$ and the broken line may be an additional carbon-carbon bond.